# EUROPEAN PATENT SPECIFICATION

(11) **EP 1 601 509 B1**
(45) Date of publication and mention of the grant of the patent: **10.11.2010**
(21) Application number: 04719256.2
(22) Date of filing: 10.03.2004
(51) Int. Cl.: B29C 59/06, A61F 13/15, B26F 1/26

(54) **METHOD OF FORMING A SOFT AND RESILIENT FILM**
VERFAHREN ZUM FORMEN EINER WEICHEN UND ELASTISCHEN FOLIE
PROCEDE POUR FORMER UN FILM SOUPLE ET ELASTIQUE

(30) Priority: 10.03.2003 US 452938 P
(43) Date of publication of application: 07.12.2005
(73) Proprietor: Tredegar Film Products Corporation, Richmond, VA 23225 (US)
(72) Inventor: COPAT, Marcelo, S., Greenwood, Indiana 43143 (US); TRIBBLE, Jim, Brazil, IN 47834 (US); SKOCHDOPOLE, Todd, R., Terre Haute, IN 47802 (US); BOWER, Sean, W., Terre Haute, IN 47803 (US); GRAY, Brian, F., Cincinnati, Ohio 45215 (US); STONE, Keith, J., Fairfield, OH 45014 (US)
(74) Representative: Finck, Dieter
(86) International application number: PCT/US2004/007262
(87) International publication number: WO 2004/080341

(56) References cited:
- EP-A2- 0 059 506
- WO-A-2004/058099
- US-A- 4 151 240
- US-A- 4 609 518
- US-B1- 6 180 052

## Description

### FIELD OF THE INVENTION

The present invention relates to a method of making a web according to the preamble of claim 1. The web may be for use in body contacting absorbent and non-absorbent articles such as baby diapers, adult incontinent articles, sanitary napkins or panty liners, facial wipes, body wipes, articles of clothing, hospital bed sheets and the like. The absorbent articles usually include a liquid pervious top sheet and a liquid impervious back sheet. At least one of the components included in at least one of the top sheet or the back sheet may comprise a web produced in accordance with the present invention. Such a method is disclosed in EP-A-059506.

### DESCRIPTION OF RELATED ART

It is known to form three-dimensional films, or formed films, by causing a molten polymer sheet to conform to the shape of a forming screen. Commonly utilized apertured formed film top sheets are disclosed in for example, Thompson, U.S. Patent Nos. 3,929,135, Mullane *et a*l., U.S. Patent 4,324,246, Radel et al., U.S. Patent 4,342,314, Raley et al., U.S. Patent No. 4,252,516, Thomas et al., U.S. Patent No 4,535,020, Junker *et al.,* U.S. Patent No. 5,591,510, U.S. Application Nos. 09/876440 filed June 6, 2001, and 60/346751 filed January 17, 2002, to Thomas and U.S. Application No. 10/082040 filed February 20, 2002, to Copat et al. The top sheets disclosed in these documents utilize vacuum to cause the polymer sheet to conform to the screen and also to cool. A preferred feature of these top sheets is that the polymer crystallizes, or "sets," after the molten polymer has been formed into a three-dimensional shape. The resultant formed film therefore is said to have "three-dirnensional memory." After formation and cooling, the film usually is wound into a roll.

When the roll is transported, particularly during summer transport and storage in trucks or non-air conditioned warehouses, web tensions and heat tend to compress the formed film causing it to temporarily lose some of its valuable three-dimensionality. The film also is compressed after it is incorporated into an article and the article is packaged. Three-dimensional memory enables the formed film to regain its original shape after the non-deforming forces are removed, usually within a few hours. This capability to resist deformation and to regain three-dimensional shape is called "resiliency." A disadvantage of these vacuum formed films is that the fiber-like wires that form the regulated pattern of apertures MAY exhibit a plastic-like surface and feeling. The plastic-like surface and feeling is viewed negatively by certain consumers that prefer nonwoven orwoven cloth-like surface and feeling.

It also is known to utilize high pressure water jet systems to cause two-dimensional polymeric webs to conform to forming structures in order to create three-dimensional webs.
WO 2004/058099 A2 discloses a two-stage forming process of macro-apertures and micro-apertures, wherein a first fluid, such as water, forms macro-apertures in a first stage by impinging a precursor web, and following the first stage, a second fluid, such as hot water or air further forms hair-like fibrils in a second stage.
U.S. Pat. Nos. 4,609,518, 4,629,643, 4,695,422, 4,778,644, and 4,839,216 to Curro (referred to herein as "the Curro patents" and "the Curro process") describe a multitude of web designs created by the combination of forming structures and high pressure water jet systems. In the Curro process, pressurized water is ejected from nozzles projecting water onto the surface of a two-dimensional, or flat, polymeric web causing the web to conform to the surface of an underlying support screen. As the screen rotates, subsequent portions of the web pass under the water jet causing subsequent portions of the web to conform to the screen. One advantage of the high pressure water systems is that the energy, or work, employed to force the un-molten web to conform to the screen is believed to soften the web, therefore water formed films are perceived as being more cloth-like in appearance and feeling than vacuum formed films.

In a preferred embodiment, Curro describes the use of a 1^{st} stage water system that utilizes a 1^{st} screen comprising micro-apertures to create micro-protuberances in the film that give the film a soft feeling texture. The micro-apertured or micro-bubbled film is useful as a back sheet. Subsequently, the film can be passed through a 2^{nd} stage water system that utilizes a 2^{nd} screen comprising macro-apertures to create macro-protuberances in the film that give the film liquid acquisition functionality. In summary, the 1^{st} stage provides softness and texture, and the 2^{nd} stage provides apertures for liquid acquisition functionality.

Films produced by the high pressure water jet systems described above are believed to have several disadvantages, including primarily poor resiliency, or stated differently, "flat film" memory. Because the precursor films used in high pressure water jet systems are made flat and then are subsequently made to conform into three-dimensional shapes, crystallization occurs while the webs are flat, and as a result the films have flat film memory. Films with flat film memory tend to regain their flat film shape over time, thereby losing the three-dimensional shape and its benefits. Another disadvantage of two-stage water jet systems is that the second stage jets spray water onto 1^{st} stage holies micro-protuberances, thereby damaging them.

Another disadvantage of the Curro process is that the 1^{st} and 2^{nd} stage system each require water to penetrate through the screen to form the apertures, thereby requiring an internal water removal system. These internal systems add complexity and cost, and increase maintenance requirements, which in turn reduces equipment utilization. Yet another disadvantage of the Curro process is that the two-stage high pressure water jet systems are expensive to operate - each stage requiring construction of a screen and procurement of high pressure water, each of which increases cost.

Where the formed films described above are used in a top sheet application, it is common to incorporate in their formulation a surfactant. The surfactant will bloom to the surface of the film over time and will render the film hydrophilic, which is a desirable property in films designed for fluid acquisition functions. One disadvantage of the two-stage water process, described in U.S. Pat. Nos. 5,834,092, 5,792,412, and 6,228,462 to Yann-Per Lee, is that a significant amount of surfactant blooms to the surface of the web before the web reaches the second stage and such surfactant is therefore washed away by the water jet. Besides the obvious problems associated with the loss of surfactant and the fact that there is an upper limit to the amount of surfactant that can be incorporated in a film, there also is the problem that surfactant washed away by the water jet builds up in the water system causing it to fail prematurely or, alternatively, requiring additional cleaning and maintenance effort. Surfactant losses are made worse by the application of hot water during the 1^{st} stage, whereby the heated water increases molecular mobility which in turn accelerate the blooming rate causing even more surfactant to wash away during the 2^{nd} stage.

One proposed solution to the above problems is the formation of three-dimensional apertures in nonwoven webs, such as, for example, by spunlacing, needle-perforation, or embossing systems. Very soft webs can be formed by these processes. However, the three-dimensional structures of such webs exhibit poor resiliency because they lack three-dimensional memory.

An alternative solution to the above problems is the lamination of nonwoven webs, apertured or unapertured, or of staple fibers, to vacuum formed films. Such combinations are described in U.S. Pat. Nos. 4,995,930, 5,591,510, 5,635,275, 5,635,276, 5,660,882, 5,698,054, 5,762,643, 5,733,628, 5,783,014, 6,242,074, and 6,303,208. Very soft and resilient webs can be formed by the lamination process described therein. However, such webs are expensive to manufacture because they require preparation of two precursor materials and their subsequent lamination.

The description herein of disadvantages associated with previous products, methods, and apparatus is in no way intended to limit the invention to embodiments that do not include these products, methods, and apparatus. Indeed, certain embodiments of the invention may include some or all of these previous products, methods, and apparatus without suffering from the disadvantages noted herein.

### SUMMARY OF THE INVENTION

The subject matter of the present invention is defined in claim 1, embodiments of which are subject of subclaims 2 to 4.

It is a feature of an embodiment of the present invention to provide an economical method for production of soft and resilient webs.

The present invention therefore is directed to an improved method for forming three-dimensional polymeric webs.

In accordance with these and other features of various embodiments of the invention, there is provided a single-stage forming, texturing and softening process for making a soft and resilient formed film. In the process of the invention, a molten polymer sheet, or a reheated precursor web, is placed in contact with an apertured rotating screen under the influence of vacuum. The apertured rotating screen includes wires that define a regulated pattern of apertures, whereby the wires are provided with texture. The apertured rotating screen and the impetus of vacuum cause the molten web to conform to the shape of the apertured rotating screen to aperture the molten sheet and, as air passes through the apertures, cause the molten web to cool, thereby obtaining three-dimensional memory. The vacuum also causes the molten web to come into contact with the textured surface of the wires causing at least partial formation of a textured pattern on the inner surface of the film. After application of vacuum and while the apertured three-dimensional web remains in close contact with the rotating screen, fluid is applied to the film to soften it and to cause it to conform even more to the textured screen wires. The film then can be removed from the screen after application of fluid, and dried if necessary.

Advantageously, a single-stage process eliminates the cost associated with a second stage, and minimizes the distance and time the web must travel before contacting fluid, thereby minimizing the amount of surfactant wash-off. Because there is no 2^{nd} water jet application stage, surfactant wash-off is reduced even more. Also advantageously, because the single screen supports both the macro-protuberances and the micro-protuberances while the fluid softening/texturing procedure takes place, the damage caused by fluid to the 1^{st} set of micro-protuberances in the Curro process is eliminated. Yet another advantage of the process of the invention is that by texturing around screen aberrations instead of micro-apertures, fluid does not have to penetrate through micro-peforations in the screen of Curro to form the texture thereby permitting use of an external water removal system. In addition to the benefits of external versus internal systems, the use of a blower system to blow-out water that may reside inside of the large vacuum formed apertures enhances dryness and permits the use of a smaller, less expensive, drying systems.

The resulting web of the invention has the resiliency of vacuum formed films and has improved softness of water formed films, when compared to prior art vacuum formed films.

### BRIEF DESCRIPTION OF THE DRAWINGS

Figure 1 is a schematic of a known vacuum forming system.

Figure 2 is a schematic of a known two-stage high pressure water jet forming system.

Figure 3 is a schematic of an embodiment of a single-stage forming system of the invention.

Figure 4 is a schematic of another embodiment of a single-stage forming system of the invention.

Figure 5 is a cross-section of a web made in accordance with the present invention.

Figure 6 is a cross-section of an absorbent article that includes a soft web of the present invention.

Figure 7 is a cross-section of an exemplary screen enveloped in etch-resist coating.

Figure 8 is a cross-section of an exemplary screen enveloped in etch-resist coating and covered with a photosensitive mask.

Figure 9 is a cross-section of an exemplary screen enveloped in etch-resist coating after metal has been etched away to expose surface aberrations.

Figure 10 is a cross-section of an exemplary screen provided with texture by an etching process.

Figure 11 is a cross-section of an exemplary film made by a known method.

Figure 12 is a cross-section of an exemplary film made by a known method.

Figure 13 is a cross-section of an exemplary film made by the novel method of the invention.

### DETAILED DESCRIPTION OF THE

### EXEMPLARY EMBODIMENTS OF THE INVENTION

Set forth below are definitions of some of the terms and expressions used herein.

The term "substantially" means that a given property or parameter (such as the surface angle) may vary by about 30% from the stated value.

The phrase "regulated pattern" means regions of protuberances in the film (and therefore in the component(s) of the absorbent article which include the film) where a selected property or selected properties of the capillaries are repeatably controlled, i.e., the property or properties is or are controlled to achieve a desired pattern of the selected property or properties. If a region comprises a regulated pattern of capillaries, it does not necessarily mean that all capillaries in that region have exactly the same property (or properties) which was selected to be controlled. It means that the selected property is varied in a designed, prescribed manner (or pattern) to substantially achieve a particular formula. Each region has only one regulated pattern.

The term "permeability" refers to the permeability of vapor or liquid. The term "coverstock" refers to the body contacting web which is part of a personal care article. Coverstock webs can be film webs, nonwovens webs, laminate webs and apertured laminate webs. The term "web" refers to a material capable of being wound into a roll. Webs can be film webs, nonwoven webs, laminate webs, apertured laminate webs etc. The term "sheet" refers to a curtain of extruded molten polymer that has not solidified into a film.

The term "film" refers to a web made by extruding a molten sheet of polymeric material by a cast or blown extrusion process and then cooling the sheet to form a solid polymeric web. Films can be monolayer films, coextruded films, coated films, and composite films. Coated films are films comprising a monolayer or coextruded film that are subsequently coated (extrusion coated, impression coated, printed) with a thin layer of the same or different material to which it is bonded, and after bonding, is incapable of separation under normal use conditions. Composite films are films comprising more than one film where the at least two films are bonded in a bonding process. Bonding processes may incorporate adhesive layers between the film layers or heat and/or pressure.

The term "fluid" is used herein to denote a flowable material, and preferably a material whose flow rate can be increased by an increase in pressure. Exemplary fluids include water, air, gels, and combinations thereof. Preferred fluids include water and air.

The term "panel" means a square or rectangularly shaped sheet of steel or similar material. The term "plate" means a bonded stack of panels. The term "perforation" as used herein refers to an aperture in the screen.

The term "indentation" as used herein refers to a recess, or debossment, located at least on the outermost surface of the screen. Indentations may connect the outermost surface and the innermost surface of the screen to create perforations in the screen. In other words, perforations are indentations but indentations are not necessarily perforations.

The phrase "inner surface" when referring to the formed film means the flat film surface that comes into contact with the screen surface. The phrase "inner surface" when referring to the screen means the screen surface that does not come into contact with the film and which is opposite the film-contacting surface of the screen. The film-contacting surface of the screen is called the "outer surface" of the screen.

The term "aberration" as used herein refers to a three-dimensional member, or embossment, with a base portion located on the outermost surface of the screen and an apex projecting outwardly from the surface of the screen. Aberrations may form regulated patterns or may be placed randomly on the screen surface.

The expression "minimum width" as used herein to describe apertures, perforations, or indentations, means the minimum distance possible between any two edges measured on the plane of the aperture (or perforation or indentation). The minimum width of a circle is its diameter. The minimum width of an ellipse is the length of its minor axis. The minimum width of a slot is the distance between its parallel long edges.

The expression "maximum width" as used herein to describe apertures, perforations, or indentations, means the maximum distance possible between any two edges measured on the plane of the aperture (or perforation or indentation). The maximum width of a circle is its diameter. The maximum width of an ellipse is the length of its major axis. The maximum width of a slot is the distance between its edges measured along a direction that is parallel to its length.

The term "mesh" as used herein to describe a regulated pattern means the square root of the maximum number of polygonal shapes (such as first apertures of protrusions) that can be inscribed in a flat square area measuring 25,4 mm (1 inch) on the side. Mesh can also be defined by describing the manner in which the protrusions are located in relation to each other as measured by their center-to-center distances. For example, a tightly packed square pattern of protuberances with 2,54 mm (0.1 inch) center-to-center distances is a 10 mesh square pattern. A tightly packed hexagonal pattern of protuberances with 2,54 mm (0.1 inch) center-to-center distances is a 10.7 mesh hexagonal pattern (hexagonal patterns being packed more tightly than square patterns). A tightly packed hexagonal pattern of protuberances with 2,54 mm (0.01 inch) center-to-center distances is a 107 mesh hexagonal pattern.

The term "screen" as used herein refers to a three-dimensional molding apparatus comprising indentations used to form protuberances in films. In a particularly preferred embodiment screens comprise tubular members, having a length and a diameter. In alternative embodiments screens comprise belts having a width and a length. The transverse direction is the direction parallel to the width of the screen. The machine direction is the direction parallel to the direction of rotation of the screen, and is perpendicular to the transverse direction.

The phrase "macro-protuberance" can be defined by reference to a three-dimensional member comprising an apertured base portion located in the plane of the first surface of the film and a sidewall portion extending generally in the direction of the second surface of the film. Each base portion has a sidewall portion. Sidewall portions terminate at an apex located in the plane of the second surface of the film. A macro-protuberance is a relatively large protuberance extending from the first surface to the second surface, and is comprised of the aperture in the base portion in the first surface, the sidewalls, and the end in the second surface. The ends of the protuberances may be apertured or unapertured. An apertured macro-protuberance also is referred to herein as a macro-aperture.

The apertures in the base portions of the protuberances, also called "primary apertures", are preferably in the shape of polygons, e.g., squares, hexagons, pentagons, ellipses, circles, ovals, slots, etc., in a regulated or random pattern. The protubered ends if apertured are called "secondary apertures", and are preferably in the shape of polygons, e.g., squares, hexagons, pentagons, ellipses, circles, ovals, slots, etc. Macro-protuberances in the film correspond to relatively large indentations in the screen, and apertured macro-protuberances in the film correspond to perforations in the screen.

The phrase "micro-protuberance" can be defined by reference to a three-dimensional member comprising an apertured base portion located in the plane of the first surface of the film and a sidewall portion extending generally in the direction of a third surface of the film. Each base portion has a sidewall portion. Sidewall portions terminate at an apex located in the plane of the third surface of the film. A micro-protuberance is a relatively small protuberance extending from the first surface to a third surface, and is comprised of an aperture in the base portion in the first surface, the sidewalls, and the end in the third surface. The ends of the micro-protuberances may be apertured or unapertured. An apertured micro-protuberance also is referred to herein as a micro-aperture. The apertures in the base portions of the micro-protuberances, also called "primary apertures", are preferably in the shape of polygons, e.g., squares, hexagons, pentagons, ellipses, circles, ovals, slots, etc., in a regulated or random pattern. Micro-protuberances in the film correspond to aberrations in the screen.

The prefix "macro" as used herein refers to structural features or elements that are readily visible to a normal human eye when the perpendicular distance between the viewer's eye and the plane of the web is about 30 centimeters (cm). Conversely, the prefix "micro" is utilized to refer to structural features or elements that are not readily visible to a normal human eye when the perpendicular distance between the viewer's eye and the plane of the web is about 30 cm.

With regard to the protubered webs of the present invention, a given protubernace's "amplitude" refers to the distance from the plane where the protuberance originates to the surface where the protuberance terminates.

As used herein, the phrase "female side" denotes the side or surface of a web where the protuberances being described originate. In contrast, the phrase "male side" denotes the side or surface of a web where the protuberances being described terminate. In the case of webs having both macro-protuberances and micro-protuberances, where the macro and micro-protuberances extend in opposite directions, the female side will be the side or surface of the web where the macro-protuberances originate, and the male side will be the side or surface of the web where the macro-protuberance terminates.

Typically, use of the phrase "micro-protuberance" refers to an protuberance having as its maximum width a distance of not more than about 300 µm, preferably not more than about 200 µm, still more preferably not more than about 100 µm. The phrase "macro-protuberance" typically refers to an aperture having a maximum width of greater than about 300 µm.

As used herein, the phrase "migratable surfactant" refers to any surfactant that is chemically incompatible with the thermoplastic polymer with which it is combined, such that it will migrate to the film's surface over time to alter the fluid handling dynamics of the polymer's surface. Representative materials useful as the surfactant and the thermoplastic polymer are described below.

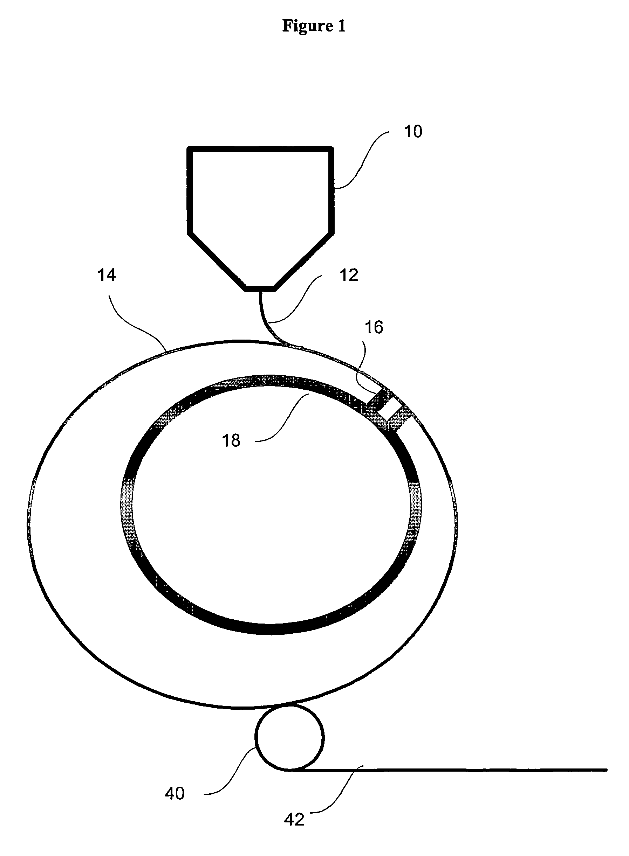

Figure 1 is a schematic of a known vacuum forming system. A polymer blend is conveyed, mixed and blended in an extruder (not shown) and delivered to the extrusion die 10, preferably a cast extrusion die 10. A molten polymer curtain 12, or sheet, exits the die and is laid over a rotating apertured screen 14. The screen conveys the polymer sheet over vacuum slot 16 where air is forced to pass through the sheet and into the vacuum tube 18 forming apertures in the film that conform to the apertures in the screen. The formed film 42 is taken off of rotating apertured screen 14 at roller 40.

Such vacuum forming systems are described in, for example, U.S. Patent Nos. 3,929,135, 4,324,246, 4,342,314, 4,252,516, 4,535,020, 5,591,510, U.S. Application Nos. 09/876440 filed June 6, 2001, 60/346751 filed January 17, 2002, and 10/082040 filed February 20, 2002. The fiber-like interconnecting wires that define the apertures in the screen may be microtextured according to the teachings of Ahr, U.S. Patent No. 4,465,045 ("Ahr"). Microtexture is defined by Ahr as aberrations (e.g., micro-protrusions) whose amplitude is about 0.3 mils (7.5 µm). The aberrations preferably are arranged in a hexagonally close packed pattern with at most 4 mil (100 µm) center-to-center spacing between adjacent aberrations. Such a regulated pattern of aberrations also can be described as a hexagonal 267 mesh pattern. As disclosed by Ahr, the microtexture can be obtained by etching the outer surface of the screen.

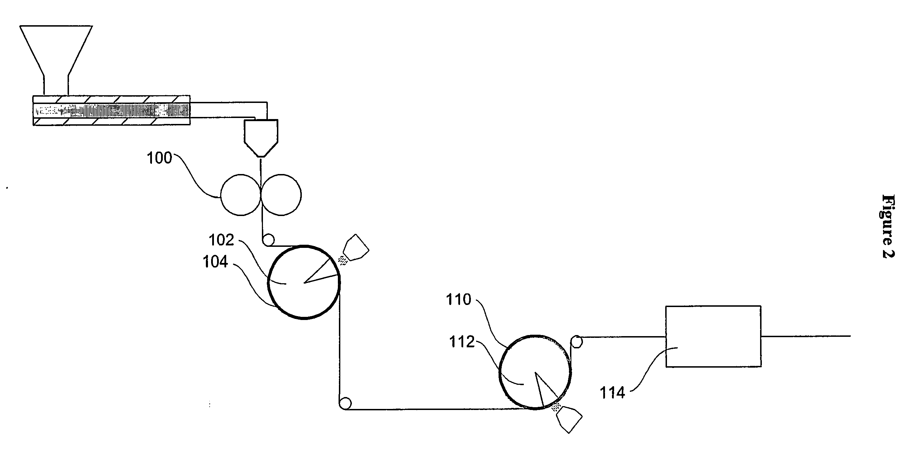

Figure 2 is a schematic of a known two-stage high pressure water forming system. A polymer blend is conveyed, mixed and blended in an extruder and delivered to the extrusion die. The molten polymer curtain, or sheet, exits the die and is laid over a rotating two roll embossing system 100 where the web is pressed and cooled and thereby acquires two-dimensional memory. The flat film is conveyed over a 1^{st} stage water forming station 102 and is caused to form micro-apertures by spraying high pressure water on the film that is supported by screen 104. Subsequently, the micro-apertured film is conveyed over a 2^{nd} stage water forming station 112 and is caused to form macro-apertures by spraying high pressure water on the film that is supported by screen 110. The formed film then is dried in drying apparatus 114.

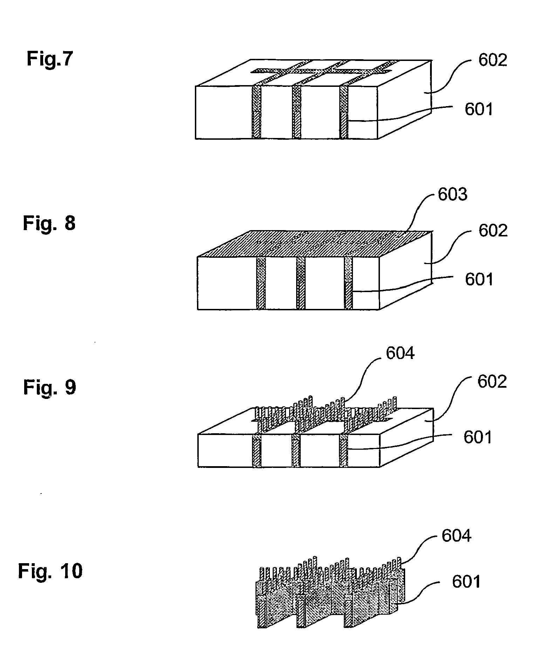

One particularly important feature of this process is that the micro-apertures terminating at a third surface formed by forcing film into micro-perforations in the screen in the 1^{st} stage extend in opposite direction as the macro apertures terminating at a 2^{nd} surface, which are formed in the 2^{nd} stage. In other words the third surface is not intermediate the first and second surfaces. Another important feature of this process is that the 2^{nd} stage forming screen provides web support to the film but it does not conform to the micro-perforation pattern of the 1^{st} stage. Accordingly, even though the web is supported, the 1^{st} stage micro-apertures are not, and as a result, the 2^{nd} stage water jet deforms the 1^{st} stage micro-apertures. This is evident clearly by the observable variation in micro-apertured diameter shown in Figure 10 of U.S. Pat. No. 6,228,462, which figure is an image of a film made by this process.

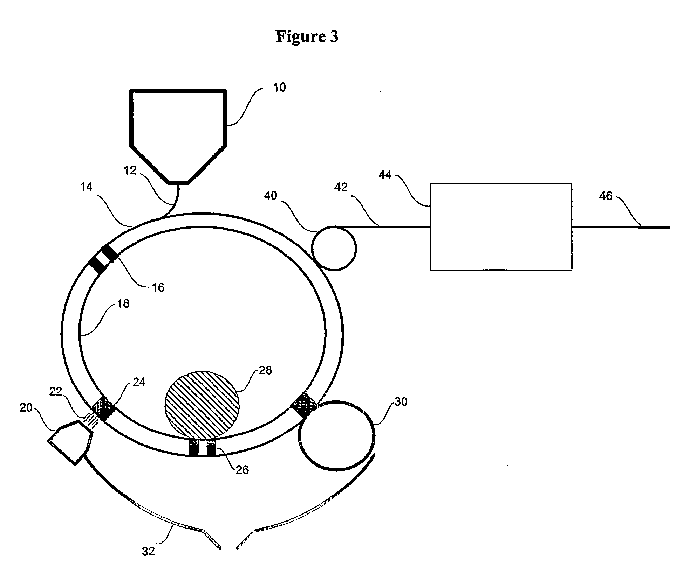

Figure 3 is a schematic of a preferred embodiment of a single-stage vacuum and fluid forming system of the invention. In general, the soft, resilient web can be made using a single rotating screen, a vacuum section, and a fluid application section. In accordance with the preferred method, a polymer blend first is conveyed, mixed and blended in an extruder by means well known in the art and therefore not shown or described in detail herein. The molten polymer can be delivered to an extrusion die 10. The molten polymer curtain 12, or sheet, exits the die and preferably although not necessarily is immediately laid over a rotating screen 14. Preferably, the screen 14 is an aperture forming/texturing screen 14. As the screen rotates the molten polymer is carried over vacuum block 16 where vacuum is applied to form macro-protuberances, and to cool the molten sheet. During this cooling process, the film crystallizes, either partially or fully, and acquires three-dimensional memory. The vacuum at vacuum block 16 can be created by suctioning air at one or both ends of vacuum tube 18, for example. Other techniques for creating a vacuum at vacuum block 16 will be readily apparent to those skilled in the art, and can be used in the context of the invention described and claimed herein.

The now resilient macro-protuberanced, preferably macro-apertured film and the screen move together to a water jet nozzle 20. A water stream 22 impinges on the film while the screen is supported by a support block 24. Applying the water through water jet nozzle 20 serves to "work" the fiber-like wires of the film to soften them, and also causes the film to adopt a mirror image of the texturing surface present in the fiber-like wires of the screen. The texture of the screen was previously impressed upon the inner surface of the film during the vacuum forming step and is now more clearly impressed such that it is more clearly visible and palpable. The additional texture enhances the perception of softness. The soft and resilient film continues to travel as the screen continues to rotate, until it passes over a blower block 26.

Air preferably is blown from one or both ends of a blown air tube 28 and exits the tube through the blower block 26 and through the macro-apertures in the soft and resilient film. As air passes through the hole it removes water droplets that might have lodged inside the apertured film protrusions. Further water removal takes place as the film passes over a support block and a water removal roller 30. Process water is captured by an external drip pan 32, enabling its easy removal and further treatment prior to disposal or recycle, if desired.

The soft and resilient film 42 then preferably is removed from the screen via removal roller 40 and dried in a drying apparatus 44. The dried soft and resilient film 46 then can subsequently be wound into a roll, if desired. Alternatively, the film 46 may be slit, corona treated, or laminated to other materials prior to winding or slitting (not shown). Alternatively the film or laminate may be die-cut and the cut components may be boxed or packaged in roll form.

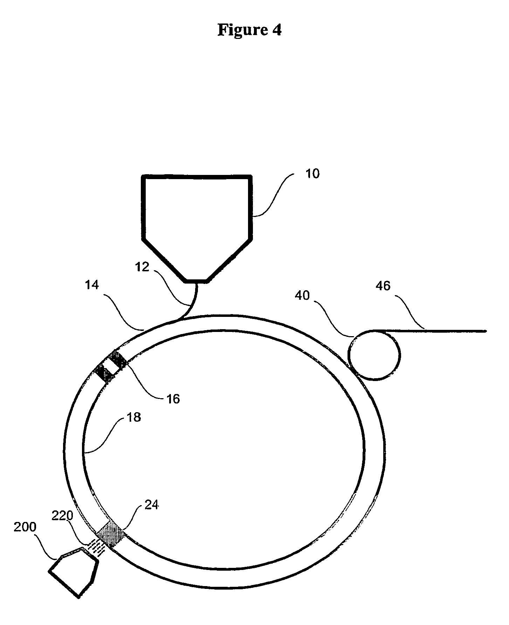

An additional preferred embodiment of the invention is illustrated in the schematic of Figure 4. As shown in this embodiment, molten polymer is delivered to an extrusion die 10 by an extruder or other mixing apparatus. The molten polymer curtain 12, or sheet, exits the die and preferably although not necessarily is immediately laid over a rotating screen 14. Preferably, the screen 14 is an aperture forming/texturing screen 14. As the screen rotates the molten polymer is carried over vacuum block 16 where vacuum is applied to form macro-protuberances, and to cool the molten sheet. During this cooling process, the film crystallizes, either partially or fully, and acquires three-dimensional memory. The vacuum at vacuum block 16 can be created by suctioning air at one or both ends of vacuum tube 18, for example.

The now resilient macro-protuberanced, preferably macro-apertured film and the screen move together to an air knife 200, or other apparatus capable of delivering pressurized air. An air stream 220 impinges on the film, preferably although not necessarily, while the screen is supported by a support block 24. Applying the air 220 through air knife 200 serves to "work" the fiber-like wires of the film to soften them, and also causes the film to adopt a mirror image of the texturing surface present in the fiber-like wires of the screen. The texture of the screen was previously impressed upon the inner surface of the film during the vacuum forming step and is now more clearly impressed such that it is more clearly visible and palpable. The additional texture enhances the perception of softness. The soft and resilient film continues to travel as the screen continues to rotate, until it proceeds to removal roller 40.

A variety of perforating screens have been developed over the years. A screen can be constructed with an electroforming technique such as that described in Pruyn, U.S. Patent 4,383,896, and in Morssinkhof, U.S. Patent 4,496,434. Briefly, in the method disclosed in Pruyn, a screen skeleton is formed on a matrix in an electrolytic bath. Thereafter the screen skeleton is stripped from the matrix and subjected to an electrolysis in a second electrolytic bath in the presence of at least one brightener. The second electrolytic bath is such that the growth of metal deposit on the screen skeleton is primarily perpendicular to the surface of the screen skeleton, whereby the metal deposited on the surface of the skeleton completes the wires that define a regulated pattern of indentations. Various metals such as copper and nickel may be used. In certain embodiments where the solid web is thicker and/or stronger (due to its composition) than other webs, and therefore where water jet energy necessary to texture the web is higher, a nickel screen will be preferred due to its additional strength, when compared to copper.

Radel, U.S. Patent 4,508,256, discloses a method of making a forming screen (the "PEL" method) by etching flat metal panels and stacking those panels in a manner such that the openings in the panels align to form passageways in the stack. The center axis of the passageways is disposed at a 90 degree angle from a plane parallel to the surface of the stack. In other words the passageways are aligned perpendicularly to the surface of the stack. The stacked panels then are bonded, rolled, and welded to form a cylindrical tube or screen. The passageways in the resulting screens also are aligned perpendicularly to the surface of the screens. Screens produced by this method are referred to as PEL screens. Rieker, U.S. Patent 5,562,932 and U.S. Patent 5,718,928, disclose an improved PEL method for producing screens capable of forming angled or curved capillaries. The Rieker patents disclose constructing the screen from multiple panels, each panel having openings that are positioned to form passageways through the screen. By stacking the panels in a manner such that the centers of the openings in each panel are offset from the centers of the openings in the remaining panels it is possible to create passageways whose center axis is disposed at an angle different than 90 degrees from a plane parallel to the surface of the screen. In other words the passageways are aligned at a non-perpendicular angle, or slanted.

Alternatively, a high energy beam perforation process can be used to form perpendicular or angled passageways in a cylinder to form a screen, for example, by the method described in Rose, WO 00/16726. The method and screen described in the Rose patent application could be improved to produce screens and to form the films and articles of the invention.

Ahr, U.S. Patent No. 4,465,045, discloses a formed film exhibiting a non-glossy visible surface. The formed film is provided with a regularly spaced microscopic pattern of surface protuberances that de-gloss the film surface. The micro-protuberances are transmitted through the thickness of the web by application of vacuum to a semi-molten web while it is in contact with a rotating screen with aberrations on its surface. The aberrations project into the film causing it to protrude on the opposite side thus forming micro-protuberances. Ahr teaches that to achieve de-glossing of the film surface the micro-protuberances must be spaced in a regulated pattern, each of the micro-protuberances in the pattern are free of planar areas that are large enough to inscribe a four mil (100 µm) diameter circle and are so spaced relative to all adjacent micro-protuberances that the maximum diameter of any circle which can be inscribed on any planar surface intermediate said micro-protuberances and said adjacent micro-protuberances is less than about four mils (100 µm).

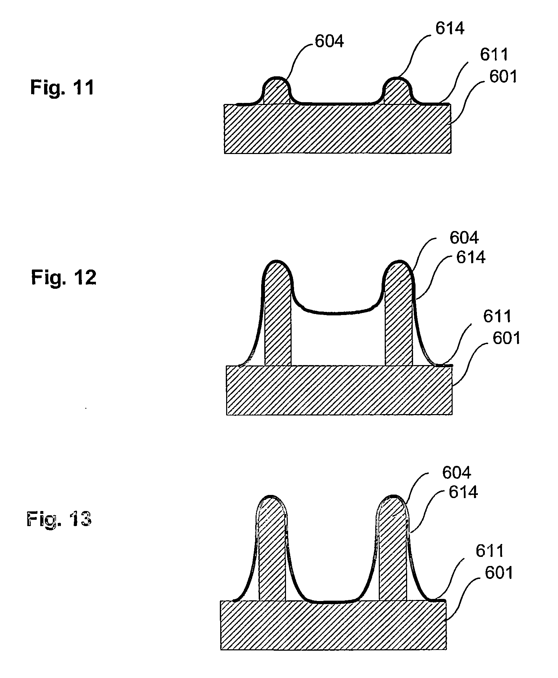

A commonly used film marketed under the trademark *"DRI-MEAVE"*® by Procter & Gamble Co. exhibits the characteristics taught by Ahr. *DRI-WEAVE*® film has a 215 mesh hexagonal pattern of micro-protuberances on its surface (the center-to-center minimum distance between any two micro-protuberances is 5 mils (125 µm)). The amplitude of such micro-protuberances is less than about 2 mils (50 µm). A cross-section of the Ahr film as it is being formed on the screen is shown in Figure 11. Aberrations 604 of cylindrical shape, about 1 mil (25 µm) in length and 1 mil (25 µm) in diameter are positioned on the surface of a perforated screen 601 on about 5 mil (125 µm) center-to-center distance. Molten polymeric film conforms to the peripheral surface of the aberration forming a micro-protuberance 614 that extends away from the planar surface of the film 611 - in other words a micro-protuberance whose third surface is not intermediate the first and second surfaces of the film where the 1^{st} surface is the planar surface and the 2^{nd} surface is the end of the macro-protuberance.

Figures 7-10 illustrate an exemplary etching process used to create a regulated pattern of aberrations on the surface of screen wires 601. The perforated screen wires 601 are enveloped in a removable etch-resist coating 602 to provide a texturing surface. The wires in the un-textured screen may be flattened by grinding or turning to provide a useful flat surface. A photosensitive mask 603 consisting of the desired texturing pattern is applied over the coating. The mask 603 and etch resist coating 602 can be designed by those skilled in the art to effect the requisite surface aberrations 604 depending on whether a positive or negative relief is formed. An illumination source is applied to cure the coating in areas not covered by the mask. The uncured coating is removed, and acid is subsequently applied to remove metal in the areas coincident with the areas where uncured coating was removed. Surface aberrations 604 are visible once the uncured coating is removed. The etch-resist coating 602 then can be removed leaving behind a finished apertured screen with surface aberrations 604. A laser process also can be utilized to provide surface aberrations. In this process a high energy beam is traced over the un-textured surface of a screen, thereby removing strips of metal of the desired depth and width. Subsequent strips can be removed until the desired surface aberrations are formed.

In an alternative method, a laser beam can be used to trace and burn-off etch-resist coating. This method eliminates the need to create and apply the mask shown in Figure 8. Those skilled in the art are capable of using any suitable method to form the screen having the dimensions described herein, using the guidelines provided herein.

The etching process controls the types of micro-protuberances that can be made. One limitation of the etching process is the depth of etching possible in relation to the etching area. The "etching aspect ratio" means the ratio of depth of etching to the minimum etching surface between unetched surfaces. For instance, a screen used to make *DRI-WEAVE*® film has a 215 mesh hexagonal aberrations pattern and the aberrations measure about 25,4 µm (1 mil) in height by 25,4 µm (1 mil) in diameter. The depth of etching is 25,4 µm (1 mil) and the minimum distance between un-etched surfaces is 101,6 µm (4 mils). The etching aspect ratio therefore is 1:4. If an aspect ratio of about 1.2:1 were used, the maximum expected etched aberration height possible for 215 mesh hexagonal patterns would be 121,9 µm (4.8 mils). Wider separation between aberrations would therefore enable the construction of greater height aberrations, and narrower separation would limit the height of the aberrations. Increasing the diameter of the aberrations will reduce their height. Using the guidelines provided in the disclosures of the Ahr, Morssinkhof, Pruyn, Rieker, Radel, and Rose patents, those skilled in the art are capable of designing a screen for use in forming films in accordance with this invention.

The soft, resilient webs of various embodiments of the present invention preferably have three-dimensional memory and the perception of softness that is desired by consumers who use the webs in body-contacting garments or articles. The webs of the embodiments of the invention have improved softness, when compared to vacuum formed films and improved resiliency, when compared to the conventional two-stage high pressure water jet made films. While not intending on being bound by any theory of operation, one reason for the improved softness is believed to be attributable to the embodiment where water is used in the second processing to help work the film (see, Figure 3).

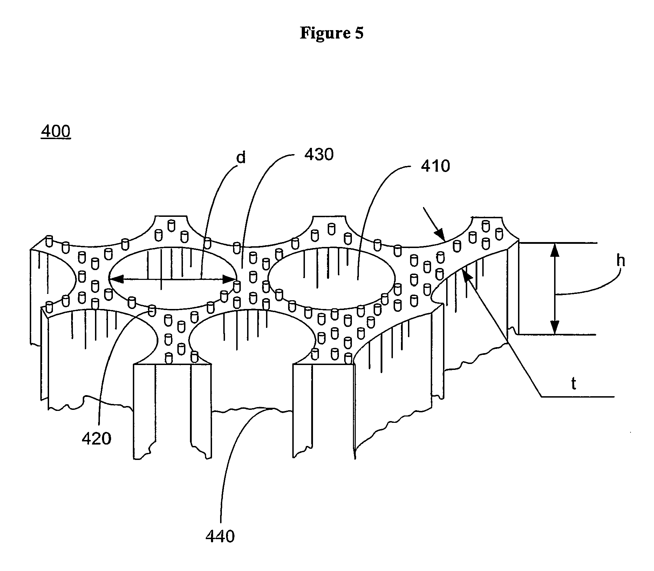

A preferred soft, resilient web of the present invention is shown in Figure 5. As shown therein, the webs 400 of embodiments of the present invention include a plurality of macro-protuberances in the form of macro-apertures 410, preferably in the shape of a pentagon, although they may have any shape and size, and a plurality of micro-protuberances 420, preferably circular in cross-section. Again, protuberances can have any cross-section. Figure 5 only shows some of the micro-protuberances 420 that may be present in web for purposes of clarity. Other commonly used shapes include hexagonal, square, and oval. Web 400 can be made from any material that is capable of being deformed by vacuum and fluid. Preferably, such deformable materials include those that, when stretched beyond their elastic limit, will substantially retain its newly formed conformation. Preferred materials for use in the invention include chemically homogeneous or heterogeneous materials such as homopolymers and polymer blends, structurally homogeneous and heterogeneous materials such as sheets and laminates, or any combinations thereof. Particularly preferred materials include polymeric films of ethylene, propylene, butylene, styrene, and butadiene, including homopolymers, copolymers, and blends thereof. Polyethylene films include high density, low density, linear low density, metallocene and conventional polyethylene polymers. As mentioned previously, web 400 may be comprised of one polymeric film, or may be a plurality of films, including co-extruded films and laminates.

The three-dimensional webs preferably have a loft, or height "h" of from about 0.100 to about 2.50 mm, preferably from about 0.100 to about 2.00 mm, and more preferably from about 0.100 to about 1.50 mm. The dimensions of the macro-apertures 410 of the invention can vary widely, depending on the desired look and feel of the web, as well as the geometric design of the apertures (i.e., hexagonal, pentagonal, heptagonal, circular, etc.). If pentagonal macro-apertures 410 are used as shown in Figure 5, the following dimensions are preferred. The thickness "t" of the ribs or wires of this particularly preferred embodiment of the invention can range anywhere from about 0.04 to about 0.80 mm, preferably from about 0.06 to about 0.70 mm, and most preferably from about 0.08 to about 0.6 mm. The diameter "d" of the macro-apertures 410, e.g., the distance from one side to the other, can be anywhere from about 0.1 to about 2.5 mm, preferably from about 0.1 to about 2.0 mm, more preferably from about 0.1 to about 1.7 mm, more preferably from about 0.1 mm to about 1.55 mm, and most preferably from about 1.55 mm to about 2.0 mm. The macro-apertures of webs of the invention will vary significantly in their structure depending on their intended use. For example *DRI-WEAYE*® film has a 25 mesh pentagonal pattern of macro-apertures where each aperture has a maximum width of about 0.8mm. Such a film is desirable for use as a top sheet or coverstock in various absorbent articles. The dimensions of the micro-protuberances 420 also will vary, as described in more detail below with reference to Figure 13.

Other films having different patterns may be produced by the method of various embodiments of the invention. For example, a hexagonal 64 mesh pattern of macro-protuberances, preferably macro-apertures, may be desirable for use as a breathable backsheet or a breathable elastic side-panel. A 64 mesh hexagonal pattern may have apertures spaced on 421,6 µm (16.6 mil) centers. Therefore, the diameters of the apertures could be controlled to provide as much or as little permeability as desired. Less permeability would require smaller apertures that correspondingly leaves more film surface to be textured. The regulated pattern of macro-protuberances also could be modified to have multiple zones of apertures, thereby providing a controlled way to impart various types of elastic properties in films. Those skilled in the art will be capable of varying any and all of the aforementioned aperture and protuberance dimensions (macro 410 and micro 420) using the guidelines provided herein, as well as the design of the web 400 (e.g., continuous array of macro-apertures 410 and micro-protuberances 420, a scattered configuration of apertures, or apertures arranged to depict a graphical image, textual message or other identifying indicia).

When used in an absorbent article, the web 400 of the present invention preferably has a body-contacting surface 430, (e.g., the first surface), and a garment or absorbent core facing surface 440 (e.g., the second surface). As shown in Figure 5, the ends of the micro-protuberances 420 terminate at a 3^{rd} surface.

Referring back to Figure 3, the preferred process of the present invention will be described in greater detail. The polymers that are to be used to form the film preferably are fed, together with any processing additives and aids known to those skilled in the art, to a melting and mixing apparatus, preferably an extruder. The molten polymer curtain 12 exiting the extrusion die 10, or a co-extruded polymer curtain 12 exiting multiple extrusion dies 10, contacts rotating aperture forming/texturing screen 14. The molten polymer curtain 12 can be fed directly to the rotating aperture forming/texturing screen 14, or it may be cooled (fully or partially) prior to such contact. Polymer curtain 12 need not be fully molten polymer, but rather can be a polymer curtain 12 that is at a temperature above the softening point of the polymer(s) used to form the curtain 12. Rotating aperture forming/texturing screen 14 can be made by any technique known in the art, and preferably is an apertured nickel electroplated screen having the desired aperture size, aberrations pattern and configuration. For example, the rotating aperture forming/texturing screen 14 can be formed into the shape shown in Figure 5.

As the screen rotates, the polymer, which remains at a temperature preferably above the softening point of the polymer(s) used to make the film, is carried over vacuum block 16 where vacuum is applied to aperture, pre-texture, and cool the molten sheet. During this cooling process, the film crystallizes, either partially or fully, and acquires three-dimensional memory. The vacuum at vacuum block 16 can be created by suctioning air at one or both ends of vacuum tube 18, for example. Other techniques for creating a vacuum at vacuum block 16 will be readily apparent to those skilled in the art, and can be used in the context of the invention described and claimed herein. The amount of vacuum created by vacuum block 16 will vary depending on the degree of deformation required, which typically is dependent upon the particular apertured configuration as well as the temperature of the polymer curtain 12. It is preferred in the present invention to direct molten polymer directly from a cast extrusion die 10 to the rotating screen 14, and that the vacuum level be from about 0 to about 500 mm Hg, more preferably from about 10 to about 250 mm Hg, and most preferably from about 20 to about 200 mm Hg. Those skilled in the art are capable of determining a suitable amount of vacuum to generate in vacuum block 16, using the guidelines provided herein.

After forming the film at the vacuum block 16, the film becomes resilient. "Resilient" as it is used herein denotes a material that substantially retains its conformation upon application of pressure and heat, with the exception of heating up to its softening temperature. Substantially retaining its conformation denotes a material that can "spring back" to up to 90%, preferably 95%, and more preferably 100% of its original conformation, after the temperature and/or pressure load have been removed. The now resilient macro-apertured film and the screen move together to a water jet nozzle 20. A water stream 22 impinges on the film while the screen is supported by a support block 24. It is preferred that the water stream be applied as a stream of water at room temperature or higher, preferably from about 40°C to about 90°C, more preferably from about 65°C to about 75°C. Preferred water pressures range anywhere from about 10 N/cm² to about 500 N/cm², more preferably from about 25 N/cm² to about 350 N/cm², and most preferably less than about 300 N/cm².

It is preferred in the present invention that water does not pass through the already formed macro-apertures 410 (Fig. 5). Applying the water through water jet nozzle 20 (Fig. 3) serves to "work" the fiber-like wires of the film to soften them, and also causes the film to adopt a mirror image of the texturing surface present in the fiber-like wires of the screen. The texture of the screen was previously impressed upon the inner surface of the film during the vacuum forming procedure and is now more clearly impressed such that it is more clearly visible and palpable. The additional texture enhances the perception of softness.

While it is preferred to use support block 24 and not to use vacuum during the water spraying procedure, support block 24 need not be used. In addition, a vacuum may be applied to assist in sucking the water through the macro-apertures 410, and to dry the apertures. In this alternative embodiment, a water removal system would be employed inside the rotating screen 14, such as those described in any of the Curro patents.

The soft and resilient film of the present invention then continues to travel as the screen 14 continues to rotate, until it preferably passes over a blower block 26. Again, although blower block 26 is preferred in the invention, a vacuum could be employed in the same or similar manner as described above. In the preferred embodiment of the invention, air is blown from one or both ends of a blown air tube 28 and exits the tube through the blower block 26 and through the macro-apertures 410 in the soft and resilient film 400. As air passes through the holes in the film, it preferably removes water droplets that might have lodged inside the apertured film protrusions. In this context, it is preferred to use heated dry air, and preferably air at a temperature within the range of from about 40°C to about 90°C, more preferably from about 65°C to about 75°C. Further water removal may optionally take place as the film passes over a support block and a water removal roller 30. Process water preferably is captured by an external drip pan 32, enabling its easy removal and further treatment prior to disposal or recycle, if desired.

The soft and resilient film 42 then preferably is removed from the screen via removal roller 40 and dried in a drying apparatus 44. The dried soft and resilient film 46 then can subsequently be wound into a roll, if desired. Alternatively, the film 46 may be slit, corona treated, or laminated to other materials prior to winding or slitting (not shown). Alternatively the film or laminate may be die-cut and the cut components may be boxed or packaged in roll form.

A cross-section of a non-performing film made by a known method is shown in Figure 12. Non-performance is primarily due to the harsh feeling texture of the film shown. Aberrations 604 of cylindrical shape, over about 2 mil (55 µm) in length and 1 mil (25 µm) in diameter are disposed on the surface of a perforated screen 601 in a 215 mesh hexagonal pattern. Molten polymeric film conforms to the peripheral surface of the aberration forming a protuberance 614 that extends away from the planar surface of the film 611. Without wishing to be bound by theory, the inventors believe that the harsh texture results because the molten polymer envelops the aberration and crystallizes without it being worked sufficiently and without it stretching sufficiently to properly form around the entire aberration and thereby causing its side-portions to thin and soften.

A cross-section of a particularly preferred film of an embodiment of the invention made by the methods described herein is shown in Figure 13. Aberrations 604 of cylindrical shape, up to about 4 mil (100 µm) in height and 1 mil (25 µm) in diameter are disposed on the surface of a perforated screen 601 in a 215 mesh hexagonal pattern. Molten polymeric film partially conforms to the peripheral surface in the presence of the first vacuum process (Fig. 3), and subsequently is caused to substantially conform, in the presence of the second fluid process, to the aberration forming a protuberance 614 that extends away from the planar surface of the film 611. The aberrations 604 of the invention preferably are from about 50,8 µm to about 127 µm (2 mils to about 5 mils) in height, and from about 25,4 µm to about 76,2 µm (about 1 to about 3 mil) in diameter, more preferably from about 25,4 µm to about 50,8 µm (about 1 to about 2 mil) in diameter, when a 215 Mesh hexagonal pattern is used. More preferably, the height of aberrations 604 is from about 63,5 µm to about 121,9 µm (about 2.5 to about 4.8 mils), and most preferably, from about 76,2 µm to about 114,3 µm (about 3.0 to about 4.5 mils), and the diameter most preferably is about 25,4 µm (1 mil). Those having ordinary skill in the art recognize how to vary the spacing between the aberrations 604, and consequently, form aberrations 604 that have greater height and/or diameter than those described above.

Webs of the invention can be made of any film forming polymers and the proper selection of polymers will depend on the desired application of the film. Films with macro-apertured patterns comprising mesh counts greater than 54 require easily flowable polymers such as low density polyethylene with high melt indeces. Low viscosity is desirable, which will also determine the choice of polymer. Elongatable polymers are desired such that films can be formed by liquid pressure around the aberrations without breaking, for example linear low density polyethylene or metallocene catalyzed linear low density polyethylene. Clearly the size and shape of the aberrations will dictate the minimum amount of elongatable polymer necessary. A person skilled in the art will recognize that various polymers can be combined to obtain the optimal formulation for each type of film produced.

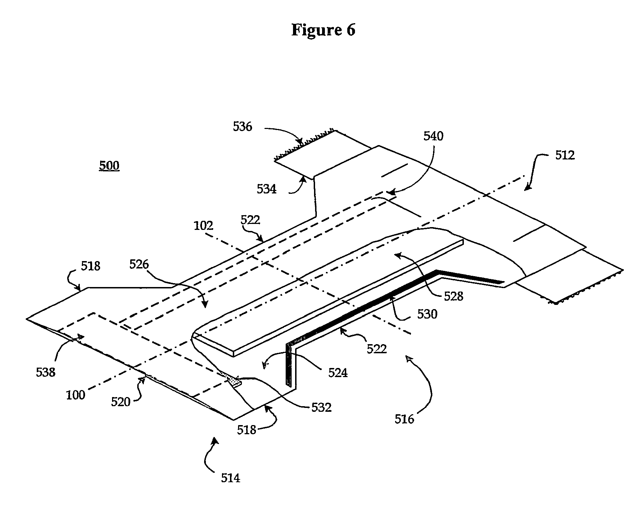

After forming film 400, the film preferably can be used as one of the elements of a body-contacting article, preferably an absorbent article. A particularly preferred absorbent article is a diaper, and a typical diaper is shown in Figure 6, with the effect of elastics removed for purposes of clarity. As used herein, the terms "absorbent garment," "absorbent article" or simply "article" or "garment" refer to devices that absorb and contain body fluids and other body exudates. More specifically, these terms refer to garments that are placed against or in proximity to the body of a wearer to absorb and contain the various exudates discharged from the body. A non-exhaustive list of examples of absorbent garments includes diapers, diaper covers, disposable diapers, training pants, feminine hygiene products, adult incontinence products, surgical gowns, wound dressing, and the like. Such garments may be intended to be discarded or partially discarded after a single use ("disposable" garments). Such garments may comprise essentially a single inseparable structure ("unitary" garments), or they may comprise replaceable inserts or other interchangeable parts.

The present invention may be used with all of the foregoing classes of absorbent garments, without limitation, whether disposable or otherwise. The embodiments described herein provide, as an exemplary structure, a diaper for an infant, however this is not intended to limit the claimed invention. The invention will be understood to encompass, without limitation, the use of the web 400 in all classes and types of absorbent garments, including those described herein.

Throughout this description, the term "disposed" and the expressions "disposed on," "disposing on," "disposed in," "disposed between" and variations thereof (e.g., a description of the article being "disposed" is interposed between the words "disposed" and "on") are intended to mean that one element can be integral with another element, or that one element can be a separate structure bonded to or placed with or placed near another element. Thus, a component that is "disposed on" an element of the absorbent garment can be formed or applied directly or indirectly to a surface of the element, formed or applied between layers of a multiple layer element, formed or applied to a substrate that is placed with or near the element, formed or applied within a layer of the element or another substrate, or other variations or combinations thereof.

Throughout this description, the terms "top sheet" and "back sheet" denote the relationship of these materials or layers with respect to the absorbent core. It is understood that additional layers may be present between the absorbent core and the top sheet and back sheet, and that additional layers and other materials may be present on the side opposite the absorbent core from either the top sheet or the back sheet.

Figure 6 is a partially cut away depiction of an exemplary embodiment of an absorbent garment 500 (preferably a disposable absorbent garment) of the present invention. The embodiment shown in Figure 6 is an infant's diaper, however, this depiction is not intended to limit the invention, and those skilled in the art appreciate that the invention covers other types of absorbent articles. For simplicity, however, the invention will be described with reference to an infant's diaper. The garment 500 of Figure 6 is depicted in a generally flattened position, with the body-facing side facing down, and with the various elastic components depicted in their relaxed condition with the effects of the elastics removed for clarity (when relaxed, the elastics typically cause the surrounding material to gather or "sliirr"). In the flattened position, the garment 500 may have a generally hourglass shaped structure, but it may also have any other shape suitable for the given application, such as a rectangular shape, a trapezoidal shape, a "T" shape, and the like.

As used herein, the longitudinal axis 100 of the garment is the dimension of the garment corresponding to the front-to-rear dimension of the user, and the lateral axis 102 of the garment is the dimension corresponding to the side-to-side dimension of the user.

In use, the invention comprises a pant-like garment 500 having a waist-encircling region and a crotch region. The waist-encircling region may comprise a first waist region 512, disposed adjacent to, for example, the back waist region of a wearer's body, and a second waist region 514, disposed adjacent to, for example, the front waist region of a wearer's body. The first and second waist regions 512, 514, may correspond to the front and back of the wearer's body, respectively, depending on whether garment 500 is attached in front of or behind the subject wearer. The first and second waist regions preferably are joined together at or near their lateral edges 518, causing the longitudinally distal edges 520 of the garment 500 to form the perimeter of a waist opening. A crotch region 516 extends between the first and second waist regions 512, 514, and the crotch edges 522 form the perimeter of a pair of leg openings, when the garment 500 is placed on a subject wearer.

The garment 500 preferably comprises a top sheet 524, and a back sheet 526, which may be substantially coterminous with the top sheet 524. When the garment 500 is being worn, the top sheet 524 faces the wearer's body, and the back sheet 526 faces away from the wearer. An absorbent core 528 preferably is disposed between at least a portion of the top sheet 524 the back sheet 526.

An embodiment of the present invention may further comprise various additional features. One or more pairs of elastic gathers 530 may extend adjacent the crotch edges 522. The garment 500 may also comprise one or more waste containment systems, such as inboard standing leg gathers 540, which preferably extend from the second waist region 514 to the first waist region 512 along opposite sides of longitudinal center line 100 (only one standing leg gather system 540 is shown in Figure 5 for purposes of clarity). One or both of the first and second waist regions 512, 514 may also be equipped with strips of elastic or foam 532 or other elastically extensible material, which help contract the garment around the wearer's waist, providing improved fit and leakage prevention.

The absorbent garment 500 also preferably includes fastening elements to enable attachment of the first waist region 512 to second waist region 514. Fastening elements preferably include a pair of tabs 534 that extend laterally away from opposite lateral edges 518 of the first waist region 512 of the garment 500. The tabs 534 may comprise an elastically extensible material (not shown), and may be designed to stretch around a wearer's waist to provide improved fit, comfort, and leakage protection. Such elasticized tabs 534 may be used in conjunction with, or in lieu of, waist foam 532, or other elastically extensible materials 532.

At least one fastening mechanism 536 (collectively referred to as "fastener 536") is attached to each tab 534 for attaching the tab to the second waist region 514, thereby providing the garment 500 with a pant-like shape, and enabling garment 500 to be fixed or otherwise fitted on the wearer. The fasteners 536 may attach to one or more target devices 538 located in the second waist region 514.

The various parts of the garment 50 can be attached to one another, associated with one another, or disposed adjacent or in between one another to form a structure that preferably maintains its shape during the useful life of the garment 500. As used herein, the terms "attached," "joined," "associated," and similar terms encompass configurations whereby a first part is directly joined to a second part by affixing the first part directly to the second part, by indirectly joining the first part to the second part through intermediate members, and by fixing the relative positions of various parts by capturing parts between other parts. Those skilled in the art will appreciate that various methods or combinations of methods may be used to securely join the respective parts of the garment 500 to one another.

The top sheet 524 and back sheet 526 may be constructed from a wide variety of materials known in the art. The invention is not intended to be limited to any specific materials for these components. The top sheet 524 and back sheet 526 can be shaped and sized according to the requirements of each of the various types of absorbent garment, or to accommodate various user sizes. In an embodiment of the invention in which the garment 500 is a diaper or an adult incontinence brief, the combination of top sheet 524 and back sheet 526, may have an hourglass shape, as seen in Figure 1, or may have a rectangular, trapezoidal, "T" shape, or other shape.

Due to the wide variety of backing and liner sheet construction and materials currently available, the invention is not intended to be limited to any specific materials or constructions of these components. The back sheet 526 preferably is made from any suitable pliable liquid-impervious material known in the art. Typical back sheet materials include films of polyethylene, polypropylene, polyester, nylon, and polyvinyl chloride and blends of these materials. For example, the back sheet can be made of a polyethylene film having a thickness in the range of 0.02-0.04 mm. The back sheet 526 may be pigmented with, for example, titanium dioxide, to provide the garment 500 with a pleasing color or to render the back sheet 526 opaque enough that exudates being contained by the garment 500 are not visible from outside the garment. In addition, the back sheet 526 may be formed in such a manner that it is opaque, for example, by using various inert components in the polymeric film and then biaxially stretching the film. Other back sheet materials will be readily apparent to those skilled in the art. The back sheet 526 preferably has sufficient liquid imperviousness to prevent any leakage of fluids. The required level of liquid imperviousness may vary between different locations on the garment 500.

The back sheet 526 may be covered with a fibrous, non woven fabric such as is disclosed, for example, in U.S. Patent 4,646,362 issued to Heran et al. Materials for such a fibrous outer liner include a spun-bonded non woven web of synthetic fibers such as polypropylene, polyethylene or polyester fibers; a non woven web of cellulosic fibers, textile fibers such as rayon fibers, cotton and the like, or a blend of cellulosic and textile fibers; a spun-bonded non woven web of synthetic fibers such as polypropylene; polyethylene or polyester fibers mixed with cellulosic, pulp fibers, or textile fibers; or melt blown thermoplastic fibers, such as macro fibers or micro fibers of polypropylene, polyethylene, polyester or other thermoplastic materials or mixtures of such thermoplastic macro fibers or micro fibers with cellulosic, pulp or textile fibers.

It is preferred in the present invention that the top sheet 524 be comprised of one or more apertured webs 400. When the apertured webs 400 are employed as the top sheet, the body-facing surface 430 (Fig. 5) of the web 400 preferably faces into the page in Figure 6 since the top sheet 524 is the lowest layer shown therein, and the garment or absorbent core facing surface 440 of web 400 preferably faces out of the page towards the absorbent core 528. Such an apertured film 400 typically is treated with a surfactant to render it hydrophilic, and as described previously, the preferred method of making the web 400 provides for less removal of the surfactant from the surface of the top sheet 524. Use of the apertured film 400 of the present invention provides a number of advantages, including the resiliency of vacuum-formed films with the improved softness of water jet formed films.

The top sheet 524 and the back sheet 526 may be associated with one another using a variety of methods known in the art. For example, they may be thermally, ultrasonically, or chemically bonded to one another. They also may be joined using lines of hot melt adhesive or mechanical fasteners, such as thread, clips, or staples. The particular joining method may be dictated by the types of materials selected for the top sheet 524 and back sheet 526.

## Claims

1. A method of making a web (400) using a single-stage vacuum and fluid forming system, the method comprising the steps of:
i. providing a web of softened material (12);
ii. depositing the web of softened material (12) on a screen (14, 601), the screen (14, 601) comprising aberrations (604) and perforations, the perforations forming on the web (400) a regulated pattern of fiber-like elements;
iii. subjecting the softened web (400) to vacuum such that macro-protuberances are formed in the web (400), the macro-protuberances forming the regulated pattern of fiber-like elements, the macro-protuberances conforming substantially to the shape of perforations in the screen (14, 601), whereby the softened web (400) is subjected to vacuum for sufficient time to cause the softened web to begin to solidify, wherein the macro-protuberances are apertured by vacuum, **characterized by**
iv. contacting the web (400) with pressurized air (220) or pressurized water (22) with sufficient energy to cause the solid web (400) to permanently deform without aperturing the web (400) in locations overlaying the aberrations (604) in the screen (14, 601) to form unapertured micro-protuberances (420); and
v. removing the web (400) from the screen (14, 601).

2. The method of claim 1, wherein an air knife (200) delivers the pressurized air.

3. The method of claim 1, wherein the aberrations in the screen have a cylindrical shape.

4. The method of claim 3, wherein the aberrations have a height within the range of from about 50.8 µm to about 127 µm (from about 2 to about 5 mils), and a diameter within the range of from about 25.4 µm to about 76.2 µm (from about 1 to about 3 mil).

## Patentansprüche

1. Verfahren zur Herstellung einer Bahn (400) unter Verwendung eines einstufigen Vakuum- und Fluidformungssystems, wobei das Verfahren die Schritte umfasst:
i. Bereitstellen einer Bahn aus weichgemachtem Material (12);
ii. Ablegen der Bahn aus weichgemachtem Material (12) auf einem Sieb (14, 601), wobei das Sieb (14, 601) Aberrationen (604) und Perforierungen umfasst, wobei die Perforierungen auf der Bahn (400) ein reguliertes Muster aus faserähnlichen Elementen bilden;
iii. Aussetzen der weichgemachten Bahn (400) einem Vakuum, so dass Makrovorsprünge in der Bahn (400) gebildet werden, wobei die Makrovorsprünge das regulierte Muster aus faserähnlichen Elementen bilden, wobei die Makrovorsprünge im Wesentlichen der Form der Perforierungen in dem Sieb (14, 601) entsprechen, wobei die weichgemachte Bahn (400) dem Vakuum über einen ausreichenden Zeitraum ausgesetzt wird, um zu verursachen, dass die weichgemachte Bahn sich zu verfestigen beginnt, wobei die Makrovorsprünge durch das Vakuum mit Öffnungen versehen werden,
**gekennzeichnet durch**
iv. In-Kontakt-Bringen der Bahn (400) mit Druckluft (220) oder Druckwasser (22) mit ausreichender Energie, um zu verursachen, dass die feste Bahn (400) sich permanent verformt, ohne die Bahn (400) an den Stellen, die über den Aberrationen (604) in dem Sieb (14, 601) liegen, mit Öffnungen zu versehen, so dass Mikrovorsprünge (420) ohne Öffnungen gebildet werden; und
v. Entfernen der Bahn (400) von dem Sieb (14, 601).

2. Verfahren nach Anspruch 1, wobei ein Luftmesser (200) die Druckluft liefert.

3. Verfahren nach Anspruch 1, wobei die Aberrationen in dem Sieb eine zylindrische Form aufweisen.

4. Verfahren nach Anspruch 3, wobei die Aberrationen eine Höhe innerhalb des Bereichs von etwa 50,8 µm bis etwa 127 µm (von etwa 2 bis etwa 5 Milli-Inch) und einen Durchmesser innerhalb des Bereichs von etwa 25,4 µm bis etwa 76,2 µm (von etwa 1 bis etwa 3 Milli-Inch) aufweisen.

## Revendications

1. Procédé de fabrication d'une bande (400) utilisant un système de formation à vide et fluide en une étape, le procédé comprenant les étapes de :
i. prévision d'une bande d'une matière ramollie (12) ;
ii. dépôt de la bande d'une matière ramollie (12) sur un écran (14, 601), l'écran (14, 601) comprenant des aberrations (604) et des perforations, les perforations formant sur la bande (400) un motif régulé d'éléments semblables à des fibres ;
iii. soumission de la bande (400) ramollie au vide de façon à ce que des macro-protubérances soient formées dans la bande (400), les macro-protubérances formant le motif régulé d'éléments semblables à des fibres, les macro-protubérances se conformant substantiellement à la forme de perforations dans l'écran (14, 601), d'où il résulte que la bande (400) ramollie est soumise au vide pendant une durée suffisante pour faire en sorte que la bande ramollie commence à se solidifier, dans lequel les macro-protubérances sont ouvertes par le vide, **caractérisé par**
iv. la mise en contact de la bande (400) avec de l'air sous pression (220) ou de l'eau sous pression (22) avec une énergie suffisante pour faire en sorte que la bande (400) solide se déforme de façon permanente sans ouverture de la bande (400) en des emplacements recouvrant les aberrations (604) dans l'écran (14, 601) pour former des micro-protubérances (420) non ouvertes ; et
v. l'enlèvement de la bande (400) de l'écran (14, 601).

2. Procédé selon la revendication 1, dans lequel une lame d'air comprimé (200) délivre l'air sous pression.

3. Procédé selon la revendication 1, dans lequel les aberrations dans l'écran ont une forme cylindrique.

4. Procédé selon la revendication 3, dans lequel les aberrations ont une hauteur dans la plage d'environ 50,8 µm à environ 127 µm (d'environ 2 à environ 5 mils), et un diamètre dans la plage d'environ 25,4 µm à environ 76,2 µm (d'environ 1 à environ 3 mils).
